(19) **Europäisches Patentamt**
**European Patent Office**
**Office européen des brevets**

(11) **EP 0 553 446 B2**

(12) **NEUE EUROPÄISCHE PATENTSCHRIFT**
Nach dem Einspruchsverfahrens

(45) Veröffentlichungstag und Bekanntmachung des
Hinweises auf die Entscheidung über den Einspruch:
**20.08.2008  Patentblatt 2008/34**

(45) Hinweis auf die Patenterteilung:
**24.03.1999  Patentblatt 1999/12**

(21) Anmeldenummer: **92120818.7**

(22) Anmeldetag: **05.12.1992**

(51) Int Cl.:
*G01N 21/88* (2006.01)          *G01N 33/36* (2006.01)
*G01N 21/89* (2006.01)          *D01H 13/22* (2006.01)
*B65H 63/06* (2006.01)

(54) **Verfahren zur Detektion von Verunreinigungen, insbesondere Fremdfasern in langgestreckten, textilen Gebilden**

Method for detecting impurities, especially foreign fibres in elongated textile articles

Procédé de détection des impuretés, notamment des fibres étrangères dans des textiles allongés

(84) Benannte Vertragsstaaten:
**BE DE FR IT**

(30) Priorität: **31.01.1992  CH 28492**

(43) Veröffentlichungstag der Anmeldung:
**04.08.1993  Patentblatt 1993/31**

(73) Patentinhaber: **Gebrüder Loepfe AG**
**CH-8623 Wetzikon (CH)**

(72) Erfinder: **Schürch, Georg**
**CH-8330 Pfäffikon (CH)**

(74) Vertreter: **Blum, Rudolf Emil et al**
**E. BLUM & Co. AG**
**Patent- und Markenanwälte VSP**
**Vorderberg 11**
**8044 Zürich (CH)**

(56) Entgegenhaltungen:
**EP-A- 0 271 728          EP-A1- 0 431 481**
**EP-A2- 0 197 763          EP-A2- 0 401 600**
**WO-A1-91/10898          WO-A1-93/13407**
**WO-A1-93/19359          CH-A- 424 316**
**CH-A- 674 379          DE-A- 1 648 430**
**GB-A- 2 095 828**

EP 0 553 446 B2

**Beschreibung**

Die Erfindung betrifft ein Verfahren und eine Vorrichtung zur Detektion von Verunreinigungen, insbesondere Fremdfasern in Garn.

Vor der Herstellung von Fäden und Garnen aus Baumwolle oder anderen Naturfasern findet zunächst eine mechanische Reinigung der Fasern durch Kardieren statt. Damit können grobe Verunreinigungen aus den Fasern entfernt werden. Anderseits gibt es gewisse Verunreinigungen, wie z.B. Fremdfasern verschiedener Art, die sich auf diese Weise nicht entfernen lassen. Diese werden in den Faden oder das Garn eingesponnen und beeinträchtigen die nachfolgenden Bearbeitungsprozesse sowie die Qualität des Endproduktes.

Bekannt sind sogenannte Garnreiniger, mit denen Garnunregelmässigkeiten wie Dick- oder Dünnstellen und dergleichen erkannt und durch Herausschneiden des betreffenden Garnstückes beseitigt werden können.

Ferner sind auch Geräte entwickelt worden, mittels denen auf optischem Weg Verunreinigungen in Garnen, z.B. sogenannte Fremdfasern, festgestellt und ebenfalls beseitigt werden können. Solche Geräte, wie sie z.B. im Europäischen Patent Nr. 197 763 oder in der Schweizer Patentschrift Nr. 674 379 beschrieben sind, beruhen im wesentlichen auf der Analyse des von einem Faden reflektierten Lichtes.

Geräte, welche nach dem optischen Prinzip arbeiten, können dabei die Fremdfasern mit Hilfe der unterschiedlichen, optischen Materialkonstanten erkennen. Die optischen Sensoren, welche mit Reflexion, Transmission und Projektion arbeiten, liefern jedoch in der Regel Signale, die auch vom Garndurchmesser abhängig sind. Deshalb wird bei den oben genannten Geräten versucht, die Anordnung so zu treffen, dass das vom optischen Sensor aufgenommene und ausgewertete Licht möglichst unabhängig vom jeweiligen Garndurchmesser ist, so dass möglichst nur Signaländerungen infolge von Fremdfasern ausgewertet werden. Solche Anordnungen sind in der Regel aufwendig und verlangen eine sehr exakte Abstimmung auf den jeweils zu bearbeitenden Faden. Damit ist es schwierig, eine ausreichende Signalunabhängigkeit von der Fadendicke zu erzielen.

Es stellt sich auf diesem Hintergrund die Aufgabe, ein Verfahren und eine Vorrichtung zum Erkennen von Verunreinigungen, insbesondere von Fremdfasern in Garnen zu schaffen, womit auf einfache Weise ein Messergebnis gewonnen werden kann, das im wesentlichen nur für das Auftreten von Verunreinigungen charakteristisch ist und nicht vom Durchmesser des geprüften Garns abhängt.

Diese Aufgabe wird durch die in den unabhängigen Ansprüchen genannten Merkmale gelöst.

Es können dabei zwei Messungen so kombiniert werden, dass der Durchmesser-Anteil der einen Messung durch die Durchmesserabhängigkeit der zweiten Messung wegkompensiert wird.

Ein üblicher Garnreinigungs-Sensor kann dafür mit einem Fremdfaser-Sensor gekoppelt werden. Der Garnreinigungs-Sensor liefert ein durchmesserproportionales Signal. Mit diesem Signal wird das Signal des Fremdfaser-Sensors verrechnet, so dass dieses beinahe nicht mehr durchmesserabhängig ist. Vorzugsweise wird diese Kompensation von einem Mikroprozessor ausgeführt.

Nachfolgend werden weitere Merkmale und Vorteile der Erfindung anhand von Ausführungsbeispielen erläutert. In den zeichnungen zeigen hierzu:

Figur 1 eine schematische Darstellung einer ersten Ausführung der Erfindung gemäß den Patentansprüchen für Be, Fr, It, nicht aber für De;
Figur 2 eine entsprechende Darstellung einer zweiten Ausführung der Erfindung.

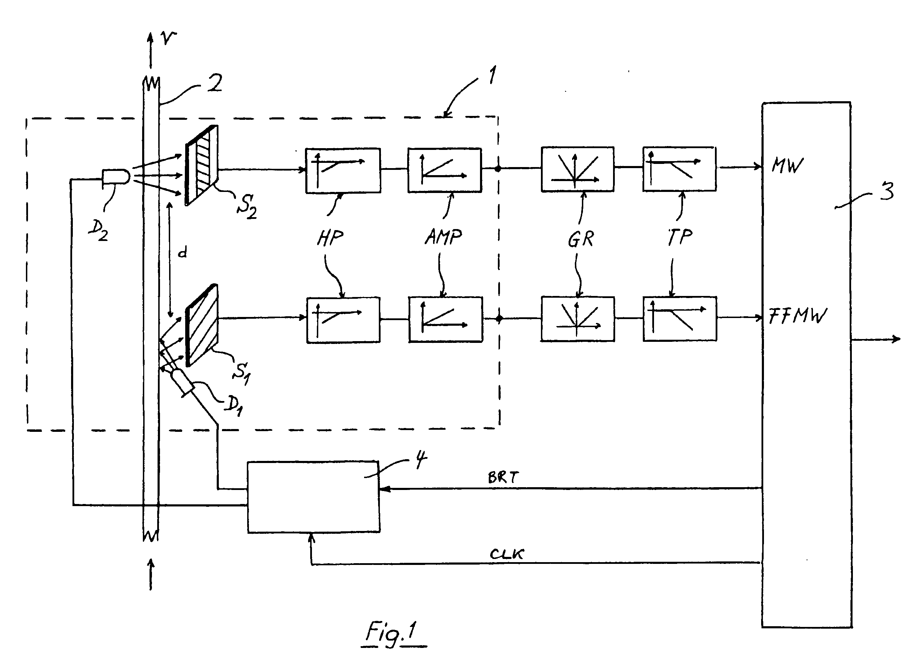

Das erste Ausführungsbeispiel, das anhand der Figur 1 erläutert wird, besitzt zwei Mess-Stellen, welche in einem gemeinsamen Sensorkopf 1 untergebracht sind, durch welchen der Faden oder das Garn 2 geführt wird. An der ersten Mess-Stelle ist ein Sensor $S_1$ angeordnet, mittels welchem gepulstes Licht aus einer Lichtquelle $D_1$ empfangen wird. Die Veränderung der empfangenen Lichtmenge und/oder die Veränderung von deren spektraler Verteilung zeigen dabei, überlagert von einem durchmesserabhängigen Anteil, das Vorliegen von Verunreinigungen an. Für den Detail-Aufbau entsprechender Mess-Stellen kann auf das Europäische Patent Nr. 197 763 oder das Schweizer Patent Nr. 674 379 verwiesen werden, wobei die dort notwendigen Abstimmungen weitgehend entfallen können. Mit Vorteil wird dabei eine Beleuchtung mittels lichtemittierenden Dioden eingesetzt, deren Wellenlänge kurzwellig, d.h. im Bereich von Grün und Blau des sichtbaren Lichtes oder im ultravioletten Bereich ist. Damit lässt sich der Kontrast und somit der Störabstand verstärken.

Das aufgenommene, pulsierende Sensor-Signal wird über einen Hochpassfilter HP und einen Verstärker AMP, die im Sensorkopf 1 angeordnet sind, sowie über einen Gleichrichter GR und einen Tiefpassfilter TP in ein Gleichstrom-Signal verwandelt, dessen Pegel in einem Mikroprozessor 3 in Digital-Signale umgewandelt wird. Die entsprechenden Messwerte werden hier als Fremdfaser-Messsignale FFMW bezeichnet. Verunreinigungen erscheinen darin als Signaländerungen, die allerdings überlagert sind von Signaländerungen, welche auf Dickenänderungen des Fadens zurückgehen.

An einer zweiten Mess-Stelle im Messkopf 1 wird ein durchmesserproportionales Mess-Signal MW aufgenommen. Das dazu verwendete Messprinzip der Projektion des mittels einer Lichtquelle $D_2$ beleuchteten Fadens 2 auf einen lichtempfindlichen Sensor $S_2$ ist aus Garnreinigern an sich bekannt und braucht deshalb nicht im Detail erläutert zu werden. Auch dieses pulsierende Sensor-Signal wird, wie oben erläutert, in ein verstärktes Gleichstrom-Signal gewandelt, dessen Pegel im Mikroprozessor 3 in Form von Digital-Signalen weiterverarbeitet wird. Dieses Signal ist im wesentlichen nur vom Durchmesser des Fadens abhängig.

Im Mikroprozessor 3 werden alsdann die jeweils zusammengehörenden Messwerte der beiden Mess-Stellen miteinander verknüpft. Bewegt sich der Faden mit einer bekannten Geschwindigkeit v und ist der Abstand zwischen den Mess-Stellen d, so bedeutet dies, dass die Messwerte der ersten Mess-Stelle mit einer Verzögerung von $\grave{U} = d/v$ mit den Messwerten der zweiten Mess-Stelle zu verknüpfen sind.

Die Verknüpfung kann nach der Formel:

$$FFMW_{KOMP} = K * \frac{FFMW}{MW}$$

erfolgen
wobei:

$FFMW_{KOMP}$ :    das durchmesserkompensierte Fremdfaser-Messsignal

$FFMW$ :    das Fremdfaser-Messsignal, wie es vom Sensor $S_1$ geliefert wird,

$MW$ :    das Mess-Signal des Reinigungssensors $S_2$, und

$K$ :    ein Koeffizient ist.

Es sind allenfalls auch andere mathematische Zusammenhänge möglich, wie z.B. geeignete Addition oder Subtraktion der Signale.

Das durchmesserkompensierte Fremdfaser-Messsignal kann hernach im Mikroprozessor 3 weiterverarbeitet werden mit dem Ziel, aus dem Messwertverlauf das Vorhandensein von Verunreinigungen abzuleiten und einen Garnreiniger entsprechend zu betätigen.

Der Mikroprozessor 3 ist neben der Auswertung des Messergebnisses auch für die getaktete Ansteuerung der lichtemittierenden Dioden verantwortlich. Hierzu liefert er einer Ansteuerschaltung 4 eine Taktfrequenz sowie ein Stellsignal zur Regelung der Helligkeit.

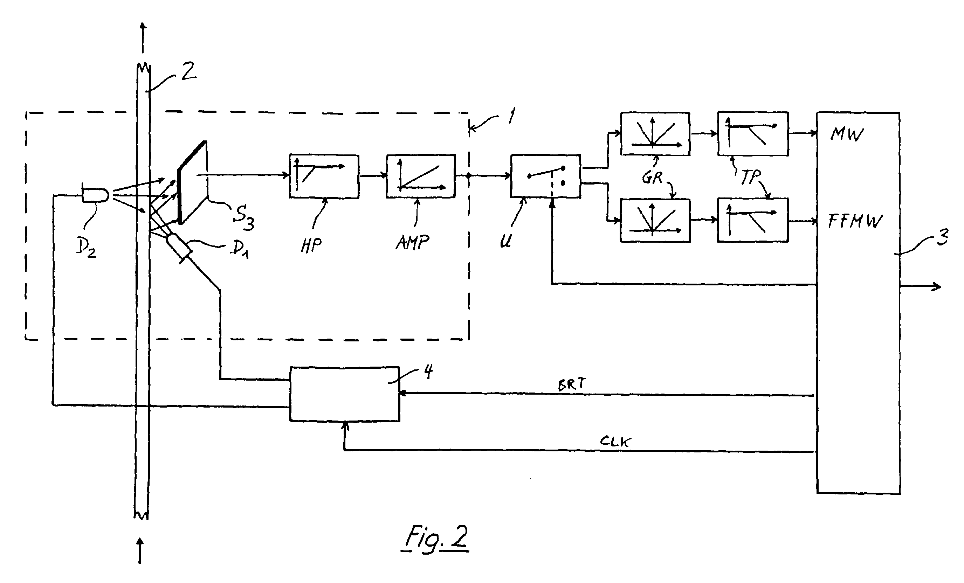

Wie bereits erwähnt, können die beiden Mess-Stellen mit Vorteil in einem einzigen Sensorkopf 1 hintereinander angeordnet werden. Statt dessen können sie auch zusammengefasst werden, wie dies anhand der Figur 2 erläutert wird. Anstelle der zwei Sensoren $S_1$ und $S_2$ tritt ein gemeinsamer Sensor $S_3$, der abwechselnd vom Faden 2 reflektiertes Licht aus der Leuchtdiode $D_1$ bzw. die Projektion des Fadens 2 mittels der Leuchtdiode $D_2$ aufnimmt. Die entsprechende Umschaltung erfolgt synchron zum alternierenden, getakteten Betrieb der beiden Leuchtdioden $D_1$ und $D_2$ durch einen Umschalter U, der vom Mikroprozessor 3 angesteuert wird. Die Ansteuerschaltung 4, welche ebenfalls vom Mikroprozessor getaktet ist, sorgt für den alternierenden Betrieb der Leuchtdioden $D_1$ und $D_2$. Die Verknüpfung der so aufgenommenen, separat zwischengespeicherten Signale erfolgt in der bereits beschriebenen Art unter Eliminierung des durchmesserabhängigen Signalanteils.

Es versteht sich, dass sowohl bei diesem als auch beim vorangehenden Ausführungsbeispiel statt jeweils einer Leuchtdiode $D_1$ bzw. $D_2$, Gruppen von entsprechenden Leuchtdioden vorgesehen sein können, wie z.B. im Europäischen Patent Nr. 197 763 beschrieben.

Erfolgt die Messung an zwei Mess-Stellen, wie im ersten Ausführungsbeispiel, so findet an einer der Mess-Stellen eine reine Durchmesserbestimmung des Fadens 2 statt, während an der anderen Mess-Stelle durch Aufnahme des reflektierten Lichtes eine mehr oder weniger durchmesserabhängige Erkennung von Verunreinigungen erfolgt. Sollen Verunreinigungen nicht nur auf der Vorderseite, sondern über den gesamten Umfang des Fadens erfasst werden, so kann dies durch Absorbtion in einem von diffusem Licht durchfluteten Messfeld geschehen, in welchem das durch den Sensor aufgenommene, vom Faden insgesamt ins Messfeld zurück reflektierte Licht messbar reduziert wird (vergl. z.B. EP-Nr. 197 763).

Die erläuterte Verknüpfung beider Messsignale erlaubt es, auf einfache Weise die Durchmesserabhängigkeit des Fremdfaser-Messsignals zu reduzieren, ohne dass hierfür ein besonderer, zusätzlicher Aufwand notwendig ist.

**Patentansprüche**

**Patentansprüche für folgende(n) Vertragsstaat(en): BE, FR, IT**

1. Verfahren zur Detektion von Verunreinigungen, insbesondere von Fremdfasern, in Garn, wobei ein erstes Signal aufgenommen wird, dessen Grösse vom Auftreten einer Verunreinigung im Garn abhängig ist und vom Durchmesser des Garns beeinflusst wird, und wobei ein zweites Signal aufgenommen wird, welches im wesentlichen nur vom Durchmesser des Garns abhängig ist, wobei das erste Signal und das zweite Signal so verknüpft werden, dass die Durchmesser-Abhängigkeit des ersten Signals im wesentlichen eliminiert wird, wobei das erste Signal im wesentlichen durch die Aufnahme von Licht erzeugt wird, das vom beleuchteten Garn reflektiert wird, und das zweite Signal im wesentlichen durch die Projektion des beleuchteten Garns auf einen lichtempfindlichen Sensor erzeugt wird.

2. Verfahren nach Anspruch 1, **dadurch gekennzeichnet, dass** die beiden jeweils miteinander verknüpften Signale im wesentlichen von derselben Stelle des Garns aufgenommen werden.

**3.** Verfahren nach einem der vorangehenden Ansprüche, **dadurch gekennzeichnet, dass** die beiden Signale an verschiedenen, voneinander beabstandeten Mess-Stellen aufgenommen werden, durch welche das Garn hindurch bewegt wird, wobei die Signale der in Bewegungsrichtung vorderen Mess-Stelle zeitlich so verzögert werden, dass sie zusammen mit den örtlich entsprechenden Signalen der anderen Mess-Stelle verknüpft werden.

**4.** Verfahren nach einem der Ansprüche 1 oder 2, **dadurch gekennzeichnet, dass** mindestens zwei alternierend betriebene Lichtquellen ($D_1$, $D_2$) und ein Sensor ($S_3$) vorgesehen sind, wobei mit dem Sensor ($S_3$) abwechselnd vom Garn reflektiertes Licht von der ersten Lichtquelle ($D_1$) bzw. eine Projektion des Garns mit der zweiten Lichtquelle ($D_2$) aufgenommen wird.

**5.** Verfahren nach einem der vorangehenden Ansprüche, **dadurch gekennzeichnet, dass** die Verknüpfung der beiden Signale durch Bildung des Quotienten des ersten mit dem zweiten Signal gebildet wird.

**6.** Verfahren nach einem der vorangehenden Ansprüche, **dadurch gekennzeichnet, dass** für die Beleuchtung des Garns gepulstes Licht mit einer Wellenlänge kleiner oder gleich grün verwendet wird.

**7.** Vorrichtung zur Detektion von Verunreinigungen, insbesondere von Fremdfasern, in Garn, **gekennzeichnet durch** mindestens einen lichtempfindlichen Sensor (S) sowie **durch** mindestens zwei Lichtquellen ($D_1$, $D_2$), wobei mindestens eine der Lichtquellen ($D_1$) das Garn so beleuchtet, dass der lichtempfindliche Sensor (S) davon reflektiertes Licht empfängt und ein erstes Signal erzeugt, und wobei mindestens eine der Lichtquellen ($D_2$) das Garn so beleuchtet, dass es auf den Sensor (S) projiziert wird, welcher daraus ein zweites Signal erzeugt, wobei die entsprechenden Sensor-Signale zur Elimination der Durchmesser-Abhängigkeit des ersten Signals in einem Rechner (3) miteinander verknüpft werden.

**8.** Vorrichtung nach Anspruch 7, **dadurch gekennzeichnet, dass** zwei Mess-Stellen ($S_1$, $D_1$; $S_2$, $D_2$) vorgesehen sind, wobei an der ersten ein lichtempfindlicher Sensor ($S_1$) zum Empfang von reflektiertem Licht des Garns angeordnet ist, und wobei an der zweiten ein lichtempfindlicher Sensor ($S_2$) vorgesehen ist, auf welchen das Garn projiziert wird.

**9.** Vorrichtung nach Anspruch 8, **dadurch gekennzeichnet, dass** an der zweiten Mess-Stelle ein Garnreiniger-Sensor ($S_2$) angeordnet ist, dessen Signale ein Mass für den Durchmesser des Garns sind und die gleichzeitig zur Ueberwachung von Dick-bzw. Dünnstellen auswertbar sind.

**10.** Vorrichtung nach einem der Ansprüche 7 bis 9, **dadurch gekennzeichnet, dass** der oder die Sensoren (S) mit einem Rechner (3) verbunden sind, in welchem die Sensor-Signale zur Kompensation der Abhängigkeit vom Durchmesser des Garns mathematisch verknüpft werden.

**11.** Vorrichtung nach einem der Ansprüche 7 bis 10, **dadurch gekennzeichnet, dass** mindestens die Lichtquellen ($D_1$), deren Licht durch Reflexion vom lichtempfindlichen Sensor (S) aufgenommen werden, Leuchtdioden mit einer Wellenlänge kleiner oder gleich grün sind.

**Patentansprüche für folgende(n) Vertragsstaat(en): DE**

**1.** Verfahren zur Detektion von Verunreinigungen, insbesondere von Fremdfasern, in Garn, wobei ein erstes Signal aufgenommen wird, dessen Grösse vom Auftreten einer Verunreinigung im Garn abhängig ist und vom Durchmesser des Garns beeinflusst wird, und dass ein zweites Signal aufgenommen wird, welches im wesentlichen nur vom Durchmesser des Garns abhängig ist, wobei das erste Signal und das zweite Signal so verknüpft werden, dass die Durchmesser-Abhängigkeit des ersten Signals im wesentlichen eliminiert wird, und wobei weiter mindestens zwei alternierend betriebene Lichtquellen ($D_1$, $D_2$) und ein Sensor ($S_3$) vorgesehen sind, wobei mit dem Sensor ($S_3$) abwechselnd vom Garn reflektiertes Licht von der ersten Lichtquelle ($D_1$) bzw. eine Projektion des Garns mit der zweiten Lichtquelle ($D_2$) aufgenommen wird.

**2.** Verfahren nach Anspruch 1, **dadurch gekennzeichnet, dass** das erste Signal im wesentlichen durch die Aufnahme von Licht erzeugt wird, das vom beleuchteten Garn reflektiert wird, und dass das zweite Signal im wesentlichen durch die Projektion des beleuchteten Garns auf einen lichtempfindlichen Sensor erzeugt wird.

**3.** Verfahren nach einem der vorangehenden Ansprüche, **dadurch gekennzeichnet, dass** die beiden jeweils miteinander verknüpften Signale im wesentlichen von derselben Stelle des Garns aufgenommen werden.

**4.** Verfahren nach einem der vorangehenden Ansprüche, **dadurch gekennzeichnet, dass** die beiden Signale an verschiedenen, voneinander beabstandeten Mess-Stellen aufgenommen werden, durch welche das Garn hindurch bewegt wird, wobei die Signale der in Bewegungsrichtung vorderen Mess-Stelle zeitlich so verzögert werden, dass sie zusammen mit den örtlich entsprechenden Signalen der anderen Mess-Stelle verknüpft werden.

5. Verfahren nach einem der vorangehenden Ansprüche, **dadurch gekennzeichnet, dass** die Verknüpfung der beiden Signale durch Bildung des Quotienten des ersten mit dem zweiten Signal gebildet wird.

6. Verfahren nach einem der vorangehenden Ansprüche 2 bis 5, **dadurch gekennzeichnet, dass** für die Beleuchtung des Garns gepulstes Licht mit einer Wellenlänge kleiner oder gleich grün verwendet wird.

7. Vorrichtung zur Detektion von Verunreinigungen, insbesondere von Fremdfasern, in Garn, **gekennzeichnet durch** genau einen lichtempfindlichen Sensor (S) sowie **durch** mindestens zwei Lichtquellen ($D_1$, $D_2$), wobei mindestens eine der Lichtquellen ($D_1$) das Garn so beleuchtet, dass der lichtempfindliche Sensor (S) davon reflektiertes Licht empfängt und ein erstes Signal erzeugt, und wobei mindestens eine der Lichtquellen ($D_2$) das Garn so beleuchtet, dass es auf den Sensor (S) projiziert wird, welcher daraus ein zweites Signal erzeugt, wobei die entsprechenden Sensor-Signale zur Elimination der Durchmesser-Abhängigkeit des ersten Signals in einem Rechner (3) miteinander verknüpft werden.

8. Vorrichtung nach Anspruch 7, **dadurch gekennzeichnet, dass** der Sensor (S) mit einem Rechner (3) verbunden ist, in welchem die Sensor-Signale zur Kompensation der Abhängigkeit vom Durchmesser des Garns mathematisch verknüpft werden.

9. Vorrichtung nach einem der Ansprüche 7 oder 8, **dadurch gekennzeichnet, dass** mindestens die Lichtquellen ($D_1$), deren Licht durch Reflexion vom lichtempfindlichen Sensor (S) aufgenommen werden, Leuchtdioden mit einer Wellenlänge kleiner oder gleich grün sind.

**Claims**

**Claims for the following Contracting State(s): DE**

1. Method for detecting impurities, in particular foreign fibres, in yarn, wherein a first signal is recorded, the magnitude of which depends on the presence of an impurity in the yarn and is influenced by the diameter of the yarn, and that a second signal is recorded, which substantially depends on the diameter of the yarn only, wherein the first signal and the second signal are combined such that the diameter dependence of the first signal is substantially eliminated, and furthermore wherein at least two alternatingly operated light sources ($D_1$, $D_2$) and a sensor ($S_3$) are provided, wherein alternatingly light from the first light source ($D_1$) reflected from the yarn or a projection of the yarn with the second light source ($D_2$) is recorded by means of the sensor ($S_3$).

2. Method of claim 1, **characterised in that** the first signal is substantially generated by recording light reflected from the yarn and that the second signal is substantially generated by projecting the illuminated yarn onto a light sensitive sensor.

3. Method of one of the preceding claims, **characterised in that** the two combined signals are recorded from substantially the same place of the yarn.

4. Method of one of the preceding claims, **characterised in that** the two signals are recorded at different measuring positions that are at a distance from each other and through which the yarn is moved, wherein the signals from the measuring position first encountered by the yarn are delayed in time in such a way that they are combined with the spatially corresponding signals from the other measuring position.

5. Method of one of the preceding claims **characterised in that** the combination of the two signals is generated by forming the quotient of the first and the second signal.

6. Method of one of the preceding claims 2 to 5, **characterised in that** pulsed light with a wavelength smaller or equal to green is used for illuminating the yarn.

**Revendications**

**Revendications pour l'(les) Etat(s) contractant(s) suivant(s): BE, FR, IT**

1. Procédé pour la détection d'impuretés et en particulier de fibres étrangères dans un fil dans lequel on enregistre un premier signal dont la grandeur dépend de la présence d'une impureté dans le fil et est influencé par le diamètre du fil, et on enregistre un second signal qui ne dépend essentiellement que du diamètre du fil, le premier et le second signal étant combinés de façon à essentiellement éliminer la dépendance du premier signal du diamètre du fil, et le premier signal étant essentiellement produit par la réception de lumière réfléchie par le fil illuminé et le second signal étant essentiellement produit par la projection du fil illuminé sur un senseur photosensible.

2. Procédé selon revendication 1 **caractérisé en ce que** les deux signaux que l'on combine sont obtenus essentiellement à partir d'un même endroit du fil.

3. Procédé selon une des revendications précédentes **caractérisé en ce que** les deux signaux sont obtenus à partir de deux champs de mesure distants l'un de l'autre et traversés par le fil, les signaux issus du champ situé en amont par rapport au déplacement étant retardés de manière à être combinés avec les signaux correspondants au même endroit du fil issus de l'autre champ de mesure.

4. Procédé selon une des revendications 1 ou 2 **caractérisé en ce que** l'on prévoit au moins deux sources lumineuses ($D_1$, $D_2$) fonctionnant alternativement et un senseur ($S_3$) recevant alternativement de la lumière issue de la première source lumineuse ($D_1$) ou une projection du fil obtenue au moyen de la seconde source lumineuse ($D_2$).

5. Procédé selon une des revendications précédentes **caractérisé en ce que** la combinaison des deux signaux est réalisée en formant le quotient du premier signal divisé par le second.

6. Procédé selon une des revendications précédentes, **caractérisé en ce que** l'on utilise pour l'illumination du fil de la lumière pulsée d'une longueur d'onde inférieure ou égale à celle du vert.

Fig.1

Fig. 2

EP 0 553 446 B2

**EP 0 553 446 B2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 197763 A
- CH 674379